# EUROPEAN PATENT APPLICATION

(11) **EP 3 701 949 A1**
(43) Date of publication of application: **02.09.2020**
(21) Application number: 18871274.9
(22) Date of filing: 21.09.2018
(51) Int. Cl.: A61K 31/4725, A61K 31/436, A61K 31/506, A61K 45/06

(54) **PHARMACEUTCAL COMPOSITION FOR PREVENTING OR TREATING CANCER, CONTAINING STREPTONIGRIN AND RAPAMYCIN AS ACTIVE INGREDIENTS**

(30) Priority: 24.10.2017 KR 20170138481
(71) Applicant: MD Biolab Co., Ltd., Seoul 02455 (KR)
(72) Inventor: KIM, Soo Youl, Goyang-si Gyeonggi-do 10382 (KR); RHA, Sun Young, Seoul 03001 (KR); BEOM, Seung Hoon, Seoul 07703 (KR); CHO, Nam Hoon, Seoul 06112 (KR); KWON, Woo Sun, Goyang-si Gyeonggi-do 10306 (KR)
(74) Representative: Schön, Christoph
(86) International application number: PCT/KR2018/011284
(87) International publication number: WO 2019/083172

(57) **Abstract**

The present invention relates to: a pharmaceutical composition for preventing or treating cancer, containing streptonigrin and an mTOR inhibitor as active ingredients; and a composition for inhibiting angiogenesis, containing streptonigrin and a vascular endothelial growth factor (VEGF) inhibitor as active ingredients. In addition, the present invention relates to: a cancer treatment method using streptonigrin and an mTOR inhibitor, a use in the preparation of anticancer drugs and a composition to be used in cancer prevention or treatment; and a method for inhibiting angiogenesis by using streptonigrin and a VEGF inhibitor, a use in the preparation of drugs for inhibiting angiogenesis and a composition to be used in angiogenesis inhibition. When a renal cancer cell line is treated with streptonigrin, which is a TGase2 inhibitor, in combination with rapamycin, which is an mTOR inhibitor, a cancer cell death effect can be exhibited to be greater than that when cancer cells are treated with each thereof, individually, and the same synergistic effect can be exhibited even in cancer cells resistant to mTOR inhibitors.

## Description

### [Technical Field]

The present application claims priority to and the benefit of Korean Patent Application No. 10-2017-0138481 filed in the Korean Intellectual Property Office on October 24, 2017, the entire contents of which are incorporated herein by reference.

The present invention relates to: a pharmaceutical composition for preventing or treating cancer, containing streptonigrin and an mTOR inhibitor as active ingredients; and a composition for inhibiting angiogenesis, containing streptonigrin and a vascular endothelial growth factor (VEGF) inhibitor as active ingredients.

In addition, the present invention relates to: a cancer treatment method using streptonigrin and an mTOR inhibitor, a use in the preparation of anticancer drugs and a composition to be used in cancer prevention or treatment; and a method for inhibiting angiogenesis by using streptonigrin and a VEGF inhibitor, a use in the preparation of drugs for inhibiting angiogenesis and a composition to be used in angiogenesis inhibition.

### [Background Art]

Cancer is a mass of cells composed of undifferentiated cells that proliferate indefinitely, ignoring the necessary state in the tissue, unlike normal cells that can be regularly and modestly proliferated and suppressed according to the needs of the individual, and is also called a tumor. These cancer cells that proliferate indefinitely are an intractable disease that invades surrounding tissues, and in more serious cases, metastasizes to other organs in the body, causing severe pain and eventually leading to death. Despite advances in medicine, it has been reported that the number of cancer cases in Korea has been steadily increasing, and thus has been increased by about 44% over the last decade, and the international market for anticancer drugs is also increasing annually, and thus is on a scale of about 100 billion dollars.

Anticancer treatments include a chemotherapeutic agent which is a first-generation anticancer drug and a targeted anticancer drug which is a second-generation anticancer drug, and in order to overcome the side effects of these, studies have been continuously conducted since an immune-anticancer drug was developed as a third-generation anticancer drug. However, the biggest problem in cancer treatment is recurrence, and since cancer-specific targets due to the diversity of cancer mutations are absent, it is known that cancer develops resistance to anticancer drugs during the treatment process, making it difficult to treat cancer, or the majority of patients die from metastatic and recurrent cancer even after treating a primary cancer. Accordingly, in order to enhance the efficacy of anticancer drugs, strategies have been suggested to mix and treat anticancer drugs in combination.

Transglutaminase 2 (TGase2) is an enzyme that promotes the binding between γ-carboxamide groups of glutamine residues bound to specific peptides and various amines and is known to play an important role in primarily the prevention of, and the promotion of defense against and recovery from damage, but recent studies have reported that abnormal overexpression may cause the occurrence of diseases such as neurodegenerative disorders, atherosclerosis, inflammatory disorders, and autoimmune disorders. In particular, it has been reported that the expression of TGase2 depletes p53 by polymerizing and destabilizing p53, and it has been reported that the inhibition of TGase2 can exhibit an anticancer effect on TGase2-overexpressing renal cancer (Patent Document 1).

Thus, the present inventors have made efforts to obtain a more effective anticancer effect in anticancer treatment using a TGase2 inhibitor, and as a result, confirmed that when cancer cells are treated with streptonigrin which is a TGase2 inhibitor which has been confirmed to be capable of being used as an anticancer drug in combination with rapamycin which is an mTOR inhibitor, a synergistic effect of anticancer activity greater than when cancer cells are treated with each alone can be exhibited thereby completing the present invention.

### [Prior Art Document]

### [Patent Documents]

(Patent Document 1) KR 10-1643459 B1 (July 21, 2016).

### [Non-Patent Documents]

(Non-Patent Document 1) Feng, Qiyu, et al. Nature Communications 8 (2017).

### [Disclosure]

### [Technical Problem]

It has been reported that it is possible to exhibit an anticancer effect against TGase2-overexpressing renal cancer by inhibiting TGase2 in the art, but since cancer develops resistance to anticancer drugs in the process of treating cancer through anticancer drugs, various side effects such as difficulties in anticancer treatment may occur, so that in order to enhance the effect of anticancer drugs, a strategy of mixing and treating anticancer drugs in combination has been suggested.

Therefore, an object of the present invention is to provide a pharmaceutical composition for preventing or treating cancer, containing a TGase2 inhibitor and an mTOR inhibitor as active ingredients.

Another object of the present invention is to provide a composition for inhibiting angiogenesis, containing a TGase2 inhibitor and a VEGF inhibitor as active ingredients.

Still another object of the present invention is to provide a cancer treatment method using a TGase2 inhibitor and an mTOR inhibitor, a use in the preparation of anticancer drugs and a composition to be used in cancer prevention or treatment.

Yet another object of the present invention is to provide a method for inhibiting angiogenesis by using a TGase2 inhibitor and a VEGF inhibitor, a use in the preparation of drugs for inhibiting angiogenesis and a composition to be used in angiogenesis inhibition.

Hereinafter, the present invention will be described in detail.

The present invention provides a pharmaceutical composition for preventing or treating cancer, containing streptonigrin and rapamycin as active ingredients.

Further, the present invention provides a pharmaceutical composition for preventing or treating cancer, further containing an anticancer drug in the aforementioned pharmaceutical composition.

In the pharmaceutical composition of the present invention, by administering the "streptonigrin" and "rapamycin" in combination, a significant synergistic effect may be obtained in cancer cell proliferation inhibition and death induction effects. In this case, it is preferred that streptonigrin and rapamycin are mixed at a molar ratio of 1 : 0.01 to 1 : 100, and specifically, it is more preferred that streptonigrin and rapamycin are mixed at a molar ratio of 1 : 0.1 to 1 : 10. From the viewpoint of significantly obtaining an anticancer effect using an anticancer drug at a low concentration through the pharmaceutical composition of the present invention, it is more preferred that the streptonigrin and the rapamycin are mixed at a molar ratio of 1 : 0.5 to 1 : 5.

In the pharmaceutical composition of the present invention, the "cancer" may be a carcinoma in which resistance to an mTOR inhibitor is induced. In this case, with respect to the mTOR inhibitor that inhibits mTOR, when TGase 2 expressed at a high level in cells blocks activity by targeting the mTOR inhibitor, the cancer may proceed to a cancer resistant to the mTOR inhibitor. The present invention may exhibit enhanced anticancer effects by treating a TGase2 inhibitor that inhibits the activity of TGase2 that blocks the activity of an mTOR inhibitor in combination with the mTOR inhibitor. Thus, the pharmaceutical composition of the present invention may exhibit a significant anticancer effect against a carcinoma in which TGase2 may be expressed to induce resistance to an mTOR inhibitor. Specifically, the carcinoma to be targeted in the present invention may be any one or more selected from the group consisting of renal cancer, breast cancer, colorectal cancer, and melanoma, and more specifically, is most preferably renal cancer, but is not limited thereto.

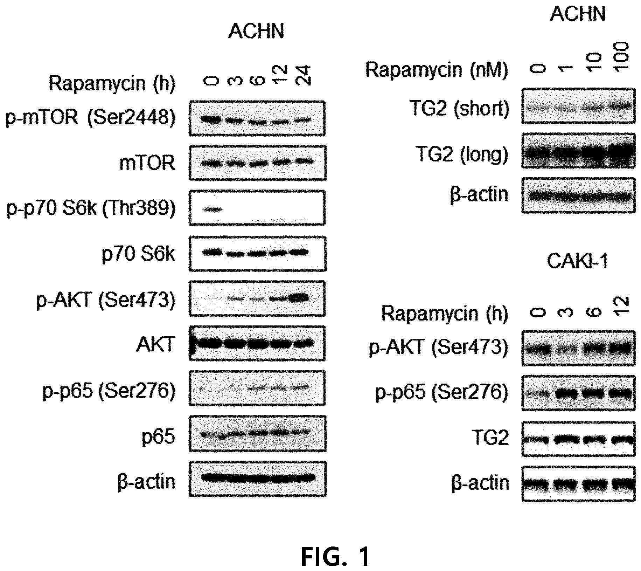

In a specific example of the present invention, the present inventors confirmed that as renal cancer that may exhibit resistance to an mTOR inhibitor was treated with rapamycin, the expression level and activity level of mTOR-related factors were decreased and the expression of TGase2 was increased (FIG. 1).

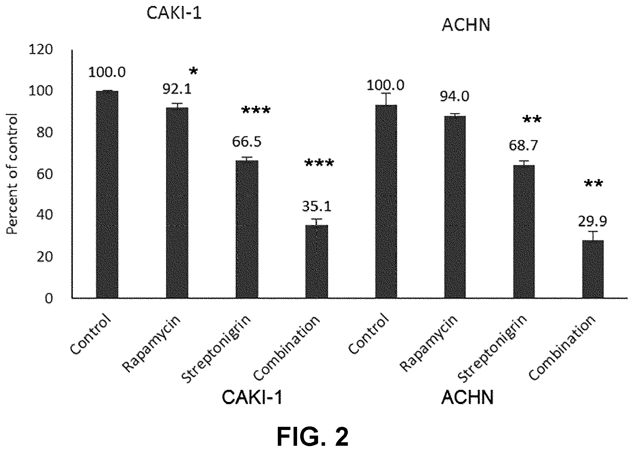

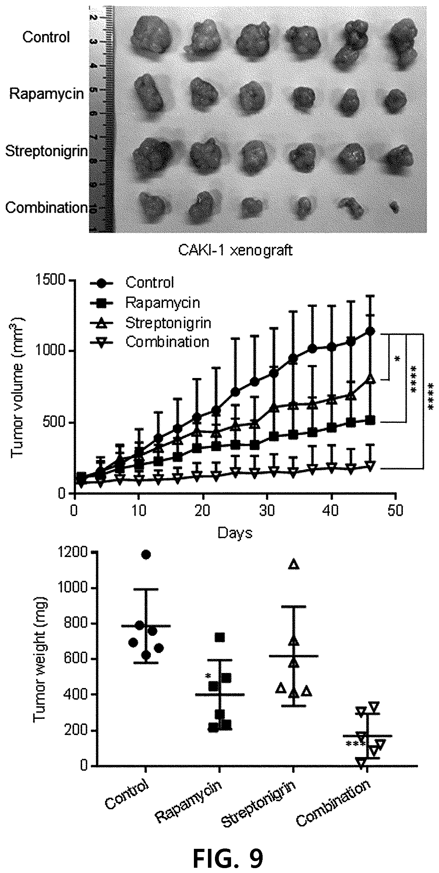

Thus, the present inventors determined that as the expression of TGase2 was increased, resistance to mTOR could be induced, and mixed rapamycin which is an mTOR inhibitor and streptonigrin which is a TGase2 inhibitor and treated a renal cancer cell line in combination with the mixture. As a result, it was confirmed that the renal cancer cell line treated with streptonigrin in combination with rapamycin exhibited significantly increased cancer cell death and tumor growth inhibition effects as compared to a single treatment group ([FIG. 2] and [FIG. 9]).

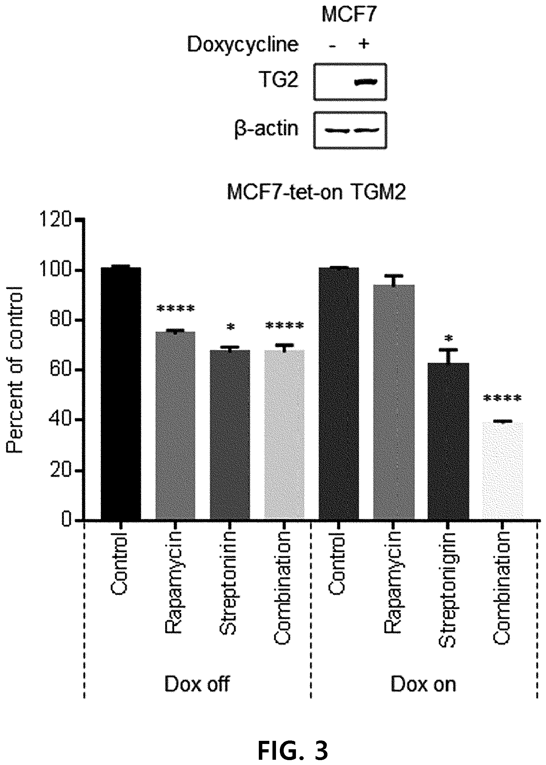

Further, the present inventors confirmed that when TGase2 was overexpressed by treating breast cancer cells with doxycycline (Dox), resistance to rapamycin was exhibited, and thus a significant cancer cell death effect failed to be exhibited in cells treated with rapamycin individually, whereas when rapamycin and streptonigrin were treated in combination, a cancer cell death effect was remarkably increased (FIG. 3).

Therefore, since the streptonigrin and rapamycin of the present invention may exhibit a significantly increased cancer cell death effect through treatment of streptonigrin and rapamycin in combination as compared to when each anticancer drug is administered individually, the pharmaceutical composition of the present invention may be usefully used in cancer prevention or treatment.

In addition, the pharmaceutical composition for preventing or treating cancer of the present invention may further contain an anticancer drug. In this case, an additionally available anticancer drug may be any one or both drugs of an anticancer drug known as an inhibitor capable of inhibiting the activity of TGase2 such as streptonigrin and used in the art and an anticancer drug known as an inhibitor that targets mTOR and used in the art, but is not limited thereto, and any anticancer drug may be used without limitation as long as the additionally available anticancer drug can be contained in the pharmaceutical composition of the present invention to exhibit enhanced anticancer activity and is within a range capable of being easily applied.

When the composition of the present invention is used as a medicine, the pharmaceutical composition containing streptonigrin and rapamycin may be formulated in various oral or parenteral administration forms as follows and administered during the clinical administration, but the administration form is not limited thereto.

Examples of a formulation for oral administration include a tablet, a pill, a hard/soft capsule, a solution, a suspension, an emulsion, a syrup, a granule, an elixir, and the like, and these formulations contains a diluent (for example: lactose, dextrose, sucrose, mannitol, sorbitol, cellulose and/or glycine) and a lubricant (for example: silica, talc, stearic acid, and a magnesium or calcium salt thereof, and/or polyethylene glycol) in addition to an active ingredient. The tablet may also contain a binder such as magnesium aluminum silicate, starch paste, gelatin, methylcellulose, sodium carboxymethylcellulose, and/or polyvinylpyrrolidine, and may contain a disintegrating agent such as starch, agar, alginic acid, or a sodium salt thereof or a boiling mixture, and/or an absorbent, a coloring agent, a flavoring agent, and a sweeting agent in some cases.

The pharmaceutical composition containing streptonigrin and rapamycin of the present invention may be parenterally administered, and the parenteral administration is performed using an injection method such as subcutaneous injection, intravenous injection, intramuscular injection, or intrathoracic injection. In this case, in order to formulate the pharmaceutical composition into a formulation for parenteral administration, the pharmaceutical composition may be prepared into a solution or suspension by mixing streptonigrin and rapamycin with a stabilizer or buffer in water and the solution or suspension may be prepared in an ampoule or vial unit administration form. The composition may be sterilized and/or contain an adjuvant such as a preservative, stabilizer, hydrating agent, or an emulsion-promoting agent, a salt and/or buffer for adjusting osmotic pressure, and other therapeutically useful materials, and the composition may be formulated using a typical method such as a mixing, granulating, or coating method.

Further, the dose of the streptonigrin and/or rapamycin of the present invention administered to the human body may vary depending on the age, body weight, gender, administration form, health status, and level of disease of a patient, and is generally 0.001 to 1,000 mg/day, preferably 0.01 to 500 mg, based on an adult patient having a body weight of 60 kg, and the composition may be administered once or in divided doses several times a day at predetermined time intervals depending on the judgment of a doctor or a pharmacist.

In addition, the present invention may provide an oral administration preparation, containing streptonigrin and rapamycin, or a pharmaceutically acceptable salt, a hydrate or a solvate thereof as active ingredients.

The oral administration preparation of the present invention may exhibit a synergistic therapeutic effect of streptonigrin and rapamycin for 60 days or more. Specifically, when streptonigrin or rapamycin is administered alone, the duration of pharmacological effects thereof is so short that an anticancer effect should be expected through repeated administration, whereas when streptonigrin and rapamycin are administered in combination, tumor growth inhibition and cancer therapeutic effects can be sustainably exhibited as compared to the case where each of streptonigrin and rapamycin is administered individually, the combination administration may be more effective than the repeated drug administration.

The oral administration preparation of the present invention may be a sustained or controlled release preparation. In the case of the sustained release preparation, streptonigrin and rapamycin may be simultaneously released, and in the case of the controlled release preparation, streptonigrin and rapamycin or rapamycin and streptonigrin may be adjusted so as to be sequentially released.

Furthermore, the present invention provides a cancer treatment method, the method including administering streptonigrin and rapamycin to a patient with cancer.

Since the streptonigrin and rapamycin are the same as those used in the pharmaceutical composition for preventing or treating cancer, the description thereof will be replaced with the above description.

Since the cancer is the same as the cancer to be prevented or treated by the pharmaceutical composition for preventing or treating cancer, the description thereof will be replaced with the above description.

Since the administration may be performed in the same manner as in the method or dosage form for administering the pharmaceutical composition for preventing or treating cancer, the description thereof will be replaced with the above description.

Further, the present invention provides a use of a composition containing streptonigrin and rapamycin in the preparation of an anticancer drug.

Since the streptonigrin and rapamycin are the same as those used in the pharmaceutical composition for preventing or treating cancer, the description thereof will be replaced with the above description.

The anticancer drug means to include all preparations that prevent or treat cancer, or inhibits metastasis.

Since the cancer is the same as the cancer to be prevented or treated by the pharmaceutical composition for preventing or treating cancer, the description thereof will be replaced with the above description.

In addition, the present invention provides a composition containing streptonigrin and rapamycin to be used in the prevention or treatment of cancer.

Since the streptonigrin and rapamycin are the same as those used in the pharmaceutical composition for preventing or treating cancer, the description thereof will be replaced with the above description.

Since the cancer is the same as the cancer to be prevented or treated by the pharmaceutical composition for preventing or treating cancer, the description thereof will be replaced with the above description.

In addition, the present invention provides a composition for inhibiting angiogenesis, containing streptonigrin and a vascular endothelial growth factor (VEGF) inhibitor as active ingredients.

Furthermore, the present invention provides a pharmaceutical composition for preventing and treating cancer or inhibiting metastasis, containing the composition for inhibiting angiogenesis.

In the pharmaceutical composition of the present invention, the "VEGF inhibitor" is more preferably pazopanib, but is not limited thereto, and may be modified and applied by the discretion of the person skilled in the art as long as the VEGF inhibitor is a drug known in the art to inhibit angiogenesis by inhibiting the expression or activity of VEGF which is an angiogenesis-inducing factor.

In the pharmaceutical composition of the present invention, by administering the "streptonigrin" and the "VEGF inhibitor" in combination, angiogenesis around tumor tissues may be effectively inhibited, and a significant synergistic effect may be obtained in cancer cell proliferation inhibition and death induction effects thereby. In this case, it is preferred that the streptonigrin and the VEGF inhibitor are mixed at a molar ratio of 1 : 1×10⁻⁵ to 1 : 1×10⁵, and specifically, it is more preferred that the streptonigrin and the VEGF inhibitor are mixed at a molar ratio of 1 : 1×10⁻⁴ to 1 : 1×10⁴. From the viewpoint of significantly obtaining an angiogenesis inhibition effect and an anticancer effect using an anticancer drug at a low concentration through the pharmaceutical composition of the present invention, it is more preferred that the streptonigrin and the VEGF inhibitor are mixed at a molar ratio of 1 : 2×10⁻⁴ to 1 : 5×10³.

In the pharmaceutical composition of the present invention, the "cancer" may be a carcinoma in which resistance to an mTOR inhibitor is induced. Specifically, the carcinoma to be targeted in the present invention may be any one or more selected from the group consisting of renal cancer, breast cancer, colorectal cancer, and melanoma, and more specifically, is most preferably renal cancer, but is not limited thereto.

Furthermore, the present invention provides a method for inhibiting angiogenesis, the method including treating streptonigrin and a vascular endothelial growth factor (VEGF) inhibitor.

Since the streptonigrin and the VEGF inhibitor are the same as those used in the pharmaceutical composition for inhibiting angiogenesis, the description thereof will be replaced with the above description.

The treatment may be performed on an individual in need of angiogenesis inhibition, such as a cow, a pig, a sheep, a chicken, a dog, and a human, and may be performed either *in vivo* or *in vitro.*

The angiogenesis may mean a disease associated with vascular cell proliferation or angiogenesis, and specifically includes ocular neovascularization, for example, retinopathy (including diabetic retinopathy), senile macular degeneration, psoriasis, angioblastoma, hemangioma, coronary sclerosis, an inflammatory disease, for example, a rheumatoid or rheumatic inflammatory disease, particularly, arthritis (including rheumatoid arthritis), or other chronic inflammatory disorders, for example, chronic asthma, arterial or post-graft coronary sclerosis, endometriosis, and neoplastic disease, for example, cancer such as a so-called solid tumor and liquid (or hematopoietic) tumor (for example: leukemia and lymphoma), and is not limited thereto. However, the angiogenesis may be cancer, and since the cancer is the same as the cancer to be targeted by the pharmaceutical composition for preventing and treating cancer or inhibiting metastasis, the description thereof will be replaced with the above description.

Further, the present invention provides a use in the preparation of a medicine for inhibiting angiogenesis using streptonigrin and a vascular endothelial growth factor (VEGF) inhibitor; and a composition to be used in cancer prevention or treatment.

Since the streptonigrin and the VEGF inhibitor are the same as those used in the pharmaceutical composition for inhibiting angiogenesis, the description thereof will be replaced with the above description.

Since the angiogenesis is the same as angiogenesis to be targeted by the method for inhibiting angiogenesis, the description thereof will be replaced with the above description.

In specific embodiments of the present invention, as a result of confirming whether the inhibition of expression or activity of TGase2 could inhibit the production of tumor blood vessels, the present inventors confirmed that when the expression or activity of TGase2 was inhibited by siRNA against TGase2 or streptonigrin, the development of blood vessel cells or the angiogenesis around cancer cells was inhibited ([FIG. 4] to [FIG. 7]). In addition, it could be confirmed that when a renal cancer cell line was treated with streptonigrin, in combination with pazopanib which is a VEGF inhibitor, a death effect against the renal cancer cell line was significantly increased as compared to the case where the cancer cell line was treated with each alone (FIG. 8).

Accordingly, the streptonigrin of the present invention may significantly inhibit the angiogenesis of tumor tissues, and accordingly, the case where the streptonigrin of the present invention is mixed with the VEGF inhibitor may inhibit the angiogenesis of tumor tissues and exhibit cancer treatment and metastasis inhibition effects while exhibiting a better synergistic effect. Therefore, the pharmaceutical composition of the present invention containing streptonigrin and a VEGF inhibitor may be usefully used for inhibiting angiogenesis, preventing and treating cancer, or inhibiting cancer metastasis.

### [Technical Solution]

To achieve the objects, the present invention provides a pharmaceutical composition for preventing or treating cancer, containing streptonigrin and rapamycin as active ingredients.

Further, the present invention provides a pharmaceutical composition for preventing or treating cancer, further containing an anticancer drug in the pharmaceutical composition.

In a preferred example of the present invention, the streptonigrin and rapamycin may be mixed at a molar ratio of 1 : 0.01 to 1 : 100.

In a preferred example of the present invention, the cancer may be a carcinoma in which resistance to an mTOR inhibitor is induced, and specifically, may be any one or more selected from the group consisting of renal cancer, breast cancer, colorectal cancer, and melanoma.

In addition, the present invention provides a composition for inhibiting angiogenesis, containing streptonigrin and a vascular endothelial growth factor (VEGF) inhibitor as active ingredients.

Furthermore, the present invention provides a pharmaceutical composition for preventing and treating cancer or inhibiting metastasis, containing the composition for inhibiting angiogenesis.

In a preferred example of the present invention, the VEGF inhibitor may be pazopanib.

In another preferred example of the present invention, the streptonigrin and the VEGF inhibitor may be mixed at a molar ratio of 1 : 1×10⁻⁵ to 1 : 1×10⁵.

In still another preferred example of the present invention, the cancer may be any one or more selected from the group consisting of renal cancer, breast cancer, colorectal cancer, and melanoma.

Furthermore, the present invention provides a cancer treatment method using streptonigrin and rapamycin, a use in the preparation of anticancer drugs and a composition to be used in cancer prevention or treatment.

In a preferred example of the present invention, the streptonigrin and rapamycin may be mixed at a molar ratio of 1 : 0.01 to 1 : 100.

In a preferred example of the present invention, the cancer may be a carcinoma in which resistance to an mTOR inhibitor is induced, and specifically, may be any one or more selected from the group consisting of renal cancer, breast cancer, colorectal cancer, and melanoma.

Further, the present invention provides a method for inhibiting angiogenesis by using streptonigrin and a vascular endothelial growth factor (VEGF) inhibitor, a use in the preparation of drugs for inhibiting angiogenesis and a composition to be used in angiogenesis inhibition.

In a preferred example of the present invention, the VEGF inhibitor may be pazopanib.

In another preferred example of the present invention, the streptonigrin and the VEGF inhibitor may be mixed at a molar ratio of 1 : 1×10⁻⁵ to 1 : 1×10⁵.

In still another preferred example of the present invention, the angiogenesis may be cancer, and specifically, may be any one or more selected from the group consisting of renal cancer, breast cancer, colorectal cancer, and melanoma.

### [Advantageous Effects]

In the present invention, it was confirmed that when a renal cancer cell line was treated streptonigrin, which is a TGase2 inhibitor, in combination with rapamycin, which is an mTOR inhibitor, a significant cancer cell death effect could be exhibited, confirming that a greater cancer cell death effect could be exhibited compared to when cancer cells are treated streptonigrin or rapamycin alone. In addition, resistance to an mTOR inhibitor was exhibited in a TGase2-overexpressing breast cancer cell line, but it was confirmed that when the breast cancer cell line was treated with streptonigrin, in combination with rapamycin, a significant cancer cell death effect could be exhibited by overcoming resistance to rapamycin.

Furthermore, in the present invention, it was confirmed that streptonigrin which is a TGase2 inhibitor could inhibit the formation of neovascular vessels around tumor tissues, and it was confirmed that when cancer cells were treated with streptonigrin, in combination with a VEGF inhibitor, a greater angiogenesis inhibition effect and a greater cancer cell death effect were exhibited compared to when cancer cells are treated with each alone.

Therefore, the present invention provides a pharmaceutical composition for preventing or treating cancer, containing streptonigrin, which is a TGase2 inhibitor and rapamycin, which is an mTOR inhibitor as active ingredients. Further, the present invention provides a composition for inhibiting angiogenesis, containing streptonigrin and a VEGF inhibitor.

### [Description of Drawings]

FIG. 1 illustrates the results of confirming expression levels of TGase2 (TG2) and related factors according to treatment time or concentration of rapamycin in renal cancer cells having mTOR resistance. As the time or concentration of rapamycin treatment was increased, the expression of TG2 and related factors was also increased, and as the color development time of the western blot was longer, a darker TG2 band was exhibited.
FIG. 2 illustrates the results of confirming the cancer cell death effect according to treatment with streptomycin or rapamycin in a renal cancer cell line. It could be confirmed that when the renal cancer cell line was treated with streptonigrin and rapamycin in combination, the death of the renal cancer cell line was significantly increased as compared to that when the renal cancer cell line was treated with streptonigrin or rapamycin alone, and particularly, an effect on an ACHN renal cancer cell line was significantly increased.
FIG. 3 is a result of confirming the synergistic effect of anticancer activity according to the treatment of a breast cancer cell line in which rapamycin resistance was induced by treating the breast cancer cell line with doxycycline (Dox) to overexpress TGase2 (T2). It could be confirmed that the death of the breast cancer cell line was significantly increased when the breast cancer cell line was treated with Dox as compared to that when the breast cancer cell line was not treated with Dox, and that the death of the breast cancer cell line was significantly increased when the breast cancer cell line was treated with streptonigrin and rapamycin in combination as compared to that when the breast cancer cell line was treated with streptonigrin or rapamycin alone.

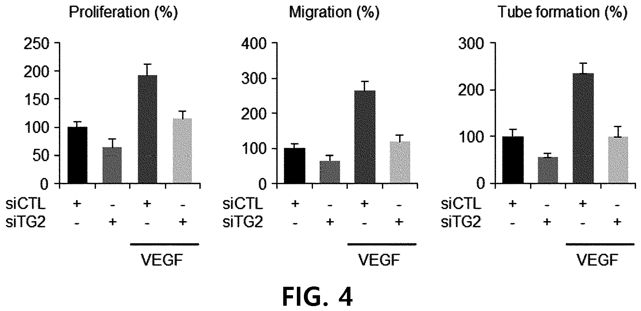

FIG. 4 illustrates the effects of inhibiting angiogenesis according to the inhibition of TGase2 expression. It could be confirmed that when blood vessel cells were treated with siTG2, the proliferation and migration of blood vessel cells (human umbilical vein epithelial cells) were inhibited, and the formation of blood vessels was also significantly inhibited, and these results were equally exhibited even in the case of treatment with VEGF which is a blood vessel growth promotion factor.

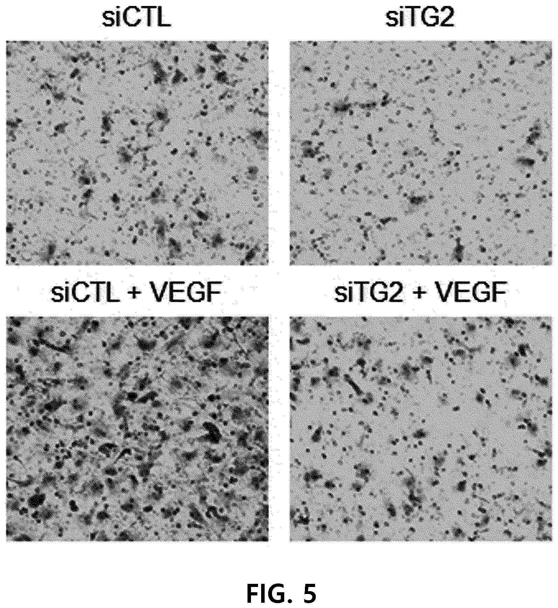

FIG. 5 illustrates the migration of blood vessel cells (human umbilical vein epithelial cells) according to treatment with siCTL, siTG2 or VEGF. In the case of treatment with siTG2, the migration of the blood vessel cells was significantly inhibited, and these results were equally exhibited even in the case of treatment with VEGF which is a blood vessel growth promotion factor.

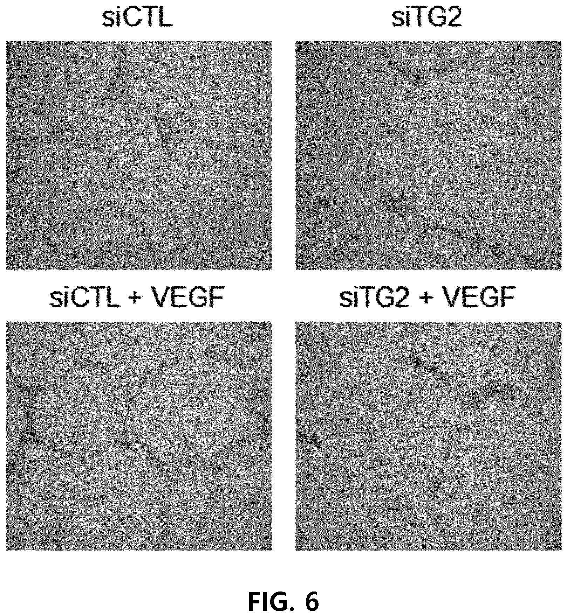

FIG. 6 illustrates angiogenesis according to treatment with siCTL, siTG2 or VEGF. In the case of treatment with siTG2, the formation of blood vessels was significantly inhibited, and these results were equally exhibited even in the case of treatment with VEGF which is a blood vessel growth promotion factor.

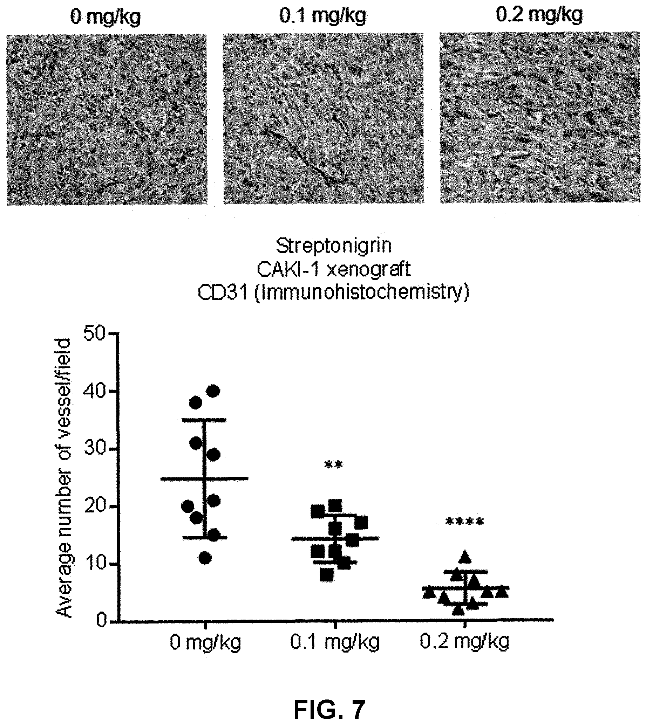

FIG. 7 is a series of results of confirming the tumor angiogenesis inhibition effect according to treatment with streptonigrin which is a TGase2 inhibitor. It could be confirmed that when renal cancer cell line (CAKI-1) xenograft mice were treated with streptonigrin, the expression of CD31 positive cells which are a blood vessel-specific marker was significantly decreased in a concentration-dependent manner.

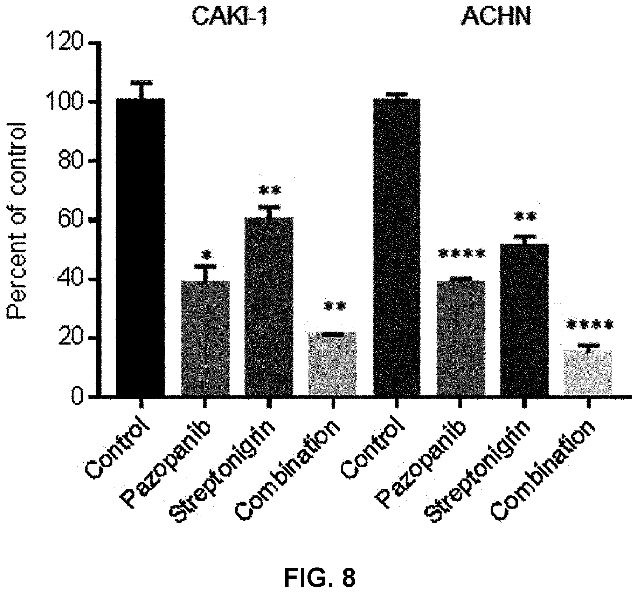

FIG. 8 illustrates cancer cell death effects according to treatment of a renal cancer cell line with streptonigrin or pazopanib. It could be confirmed that when the renal cancer cell line was treated with streptonigrin and pazopanib in combination, the death effect on the renal cancer cell line was significantly increased as compared to that when the renal cancer cell line was treated with streptonigrin or pazopanib alone.
FIG. 9 illustrates tumor growth inhibition effects when renal cancer cell line (CAKI-1) xenograft mice were treated with streptonigrin or rapamycin. It could be confirmed that when the tumor was treated with streptonigrin and rapamycin in combination, the volume and weight of a tumor were significantly decreased as compared to those when the tumor was treated with streptonigrin or rapamycin alone.

### [Modes of the Invention]

Hereinafter, the present invention will be described in more detail through Examples. These Examples are only for exemplifying the present invention, and it should be obvious to a person with ordinary skill in the art that the scope of the present invention is not interpreted as being limited by these Examples.

### Example 1. Confirmation of relationship between TGase2 and mTOR-resistant cancer

The present inventors confirmed that through previous studies, the overexpression of TGase2 promoted cancer growth, induced resistance to anticancer drug and radiation therapy, promoted cancer angiogenesis, and inhibited the death of cancer cells, and accordingly, confirmed that an inhibitor which inhibited the activity of TGase2 could inhibit the recurrence of cancer (Patent Document 1). Further, since resistance to various anticancer drugs may be induced by the activation of mTORC2 and NF-κB, it was confirmed in the present invention whether the expression and activity levels of TGase2 (TG2) were changed when cancer cells were treated with rapamycin known as an mTOR inhibitor.

Specifically, after ACHN cells which are a renal cancer cell (RCC) line and CAKI-1 cells (provided by the National Cancer Center, Korea) were each inoculated into an RPM1640 medium containing 10% FBS and cultured, and then further cultured after being treated with rapamycin. In this case, in order to confirm the change in TGase2 according to rapamycin treatment time, cells were respectively obtained at 0, 3, 6, 12, 24 hours after the treatment with rapamycin at a concentration of 10 nM was initiated; and in order to confirm the change in TGase2 according to rapamycin treatment concentration, cells that were respectively treated at a concentration of 0, 1, 10, and 100 nM and additionally cultured for 24 hours were obtained. A supernatant obtained by crushing the respectively obtained cells was obtained as a protein extract, and then the expression level of a target protein was confirmed by performing the western blot. The expression of the target protein was confirmed using each of an anti p-mTOR(Ser2448) antibody, an anti-mTOR antibody, an anti-p-p70 S6k(Thr389) antibody, an anti-p70 S6k antibody, an anti-p-AKT(Ser473) antibody, an anti-p-AKT antibody, an anti-p-p65(Ser276) antibody, an anti-p65 antibody, an anti-TGase2 antibody, and an antibody-β-actin antibody as a primary antibody for performing the western blot.

As a result, as illustrated in [FIG. 1], it was confirmed that the activity levels of mTOR-related factors were changed according to the rapamycin treatment time of renal cancer cells. It was confirmed that the p-mTOR was decreased and p-p70 S6k was inhibited by treatment with rapamycin, whereas p-AKT and p-p65 were increased, and thus activated. At the same time, it was confirmed that as renal cancer cells were treated with rapamycin, p-p65(NF-κB) was activated, and TG2 was also increased. Further, it could be confirmed that the expression or activity of TG2 was increased depending on the rapamycin treatment concentration, and the longer the color development time was during the process of the western blot, the darker the color of the TG2 band. Through this, it can be seen that TGase2 may cause mTOR resistance.

### Example 2. Confirmation of death effect on cancer cell line according to combination treatment with TGase2 inhibitor and mTOR inhibitor

### <2-1> Confirmation of renal cancer cell line death effect according to combination treatment with streptonigrin and rapamycin

After confirming that TGase2 may cause mTOR resistance in cancer cells, in order to confirm whether the anticancer effect could be increased when cancer cells were treated with a TGase2 inhibitor and an mTOR inhibitor in combination, streptonigrin which is a TGase2 inhibitor and rapamycin which is an mTOR inhibitor, were mixed and cancer cells were treated with the resulting mixture.

Specifically, after ACHN cells which are a renal cancer cell (RCC) line and CAKI-1 cells (provided by the National Cancer Center, Korea) were each inoculated into an RPM1640 medium containing 10% FBS and cultured, and then further cultured for 48 hours after being treated with 10 nM rapamycin and/or 10 nM streptonigrin in combination or alone. After the culture, cells were stained by treating respective cells with 0.4% trypan blue, and live and dead cells were sorted and counted. For the number of counted cells, the ratio of the number of relative live cells was calculated based on the number of live cells of an untreated control which had not been treated with rapamycin and streptonigrin.

As a result, as illustrated in [FIG. 2], a significantly increased cancer cell death level was confirmed in renal cancer cells treated in combination by mixing streptonigrin and rapamycin . It was confirmed that as compared to the untreated control which had not been treated with the anticancer drugs, a renal cancer cell line treated with rapamycin individually exhibited 90% or more cell viability, and a renal cancer cell line treated with streptonigrin individually exhibited 65% or more cell viability. In contrast, when treated with rapamycin and streptonigrin in combination, CAKI-1 cells and ACHN cells exhibited about 35% cell viability and about 30% cell viability, respectively, so that it was confirmed that the combination treatment group exhibited a remarkably increased cell death effect as compared to experimental groups to which rapamycin and streptonigrin were administered alone.

### <2-2> Confirmation of synergistic anticancer effect of streptonigrin on rapamycin resistance-induced cancer cell line

It is known that TGase2 is expressed at a higher level in a renal cancer cell line than in other cells, and it was confirmed that when streptonigrin and rapamycin were administered in combination to these renal cancer cell lines, a synergistic effect of anticancer activity could be exhibited. Thus, it was confirmed whether a change in anticancer effect could be exhibited according to the treatment of a cancer cell line in which rapamycin resistance was induced due to resistance to mTOR with a TGase2 inhibitor.

Specifically, MCF7 cells which are a breast cancer cell line (provided by the National Cancer Center, Korea) were inoculated into an RPM1640 medium containing 10% FBS and cultured, and then the overexpression of TGase2 was induced by treating the cultured cells with 10 nM doxycycline (Dox). For the overexpression of TGase2, the overexpression of TGase 2 according to the presence or absence of treatment with Dox was confirmed by performing the western blot using an anti-TGase2 antibody. And then, MCF7 cells in which the overexpression of TGase2 was induced and MCF7 cells which were not treated with Dox were treated with 10 nM rapamycin and/or 10 nM streptonigrin, in combination or alone, and then further cultured for 48 hours. After the culture, cells were stained by treating respective cells with 0.4% trypan blue, and live and dead cells were sorted and counted. For the number of counted cells, the ratio of the number of relative live cells was calculated based on the number of live cells of an untreated control which had not been treated with rapamycin and streptonigrin.

As a result, as illustrated in [FIG. 3], it was confirmed that TGase2 was significantly overexpressed in a breast cancer cell line (MCF7 cell) treated with Dox. First, a significant cancer cell death effect was exhibited when MCF7 cells which had not been treated with Dox were treated with rapamycin alone whereas in MCF7 cells which had been treated with Dox, resistance to rapamycin was induced along with the overexpression of TGase2, so that when treated with rapamycin, the cancer cell death effect was not significantly exhibited. When streptonigrin was administered alone, it was confirmed that a similar level of the cell death effect was exhibited regardless of the overexpression of TGase2 by confirming that a similar level of the cell death effect was exhibited regardless of the treatment with Dox. In contrast, it was confirmed that when rapamycin and streptonigrin were administered in combination, a remarkably increased cancer cell death effect was exhibited in an MCF7 cell line in which rapamycin resistance occurred by inducing the overexpression of TGase2 than that in an MCF7 cell population in which the overexpression of TGase2 was not induced (not treated with Dox).

That is, even though resistance to rapamycin was induced in a breast cancer cell line in which the overexpression of TGase2 was induced by treatment with Dox, it was confirmed that when rapamycin and streptonigrin were administered in combination, the cancer cell death effect was remarkably increased by exhibiting a synergistic effect of anticancer activity.

### Example 3. Confirmation of effect of inhibiting tumor angiogenesis according to treatment with TGase2 inhibitor and VEGF inhibitor in combination

### <3-1> Confirmation of whether angiogenesis is inhibited according to inhibition of TGase2 expression

According to reported studies, TGase2 may serve to promote the growth and metastasis of a tumor by promoting angiogenesis (Non-Patent Document 1). Thus, the present inventors intended to first confirm whether angiogenesis was inhibited according to the inhibition of TGase2 expression by confirming whether the TGase2 inhibitor of the present invention could serve to inhibit the production of tumor blood vessels.

Specifically, human umbilical vein endothelial cells (HUVECs) were inoculated into a medium and cultured, and then further cultured after the medium was treated with siRNA of siCTL or siTGase2 (siTG2) and VEGF as a vascular growth promoting factor. After cells treated with the siRNA and VEGF were cultured, the medium was again treated with bromodeoxyuridine (BudUrd), a BrdUrd labeling assay was performed. For quantification, BrdUrd-positive cells were counted among the cells grown on the medium. For the counted cells, the number of BrdUrd-positive cells in the siCTL-treated and VEGF-untreated groups was used as a reference, and the number of BrdUrd cells in each experimental group was obtained as a relative percentage.

At the same time, an angiogenesis analysis was also performed. Cells were allowed to adhere to the well surface and cultured by inoculating HUVEC cells into Matrigel-coated wells. Cells were further cultured by adding VEGF and each siRNA to the medium, and then tube segments formed were counted using an angiogenesis assay kit.

As a result, as illustrated in [FIG. 4] to [FIG. 6], when siCTL-treated cells used as a control were treated with VEGF which is an angiogenic factor, all of the proliferation, migration, and angiogenesis degrees of cells were sharply increased. In contrast, it could be confirmed that in cells treated with siTG2, the proliferation, migration, and angiogenesis degrees of cells were significantly decreased as compared to the control, and particularly, the degrees to which the proliferation, migration and angiogenesis of cells were increased by treatment with VEGF were decreased by 50% or more as compared to the control. The data indicates that when the expression of TGase2 is inhibited, angiogenesis may be inhibited in a cell population.

### <3-2> Confirmation of effect of inhibiting tumor angiogenesis according to treatment with streptonigrin

After confirming that angiogenesis could be inhibited when the expression of TGase2 was inhibited, it was confirmed whether angiogenesis around cancer cells could be inhibited in the case of treatment with streptonigrin which is a TGase2 inhibitor.

Specifically, a mouse model was prepared by xenografting mice with a CAKI-1 cell line which is a renal cancer cell line. And then, the formation of a tumor was observed after administering streptonigrin to the mouse model at a dose of 0.1 mg/kg or 0.2 mg/kg, and breeding the mice. After the xenografting, a biopsy tissue sample was obtained by excising tumor sites, and the level of expression of CD31-positive cells, which are a blood vessel specific marker, was confirmed by immunohistochemically staining the sample.

As a result, as illustrated in [FIG. 7], as the dose of streptonigrin was increased, the level of the number of CD31-positive cells was decreased, so that it was confirmed that in an experimental group to which streptonigrin was administered at a dose of 0.2 mg/kg, a level of CD31-positive cells at about 20% of a control to which streptonigrin was not administered was exhibited. Through this, it was confirmed that as the activity of TGase2 was inhibited by treatment with streptonigrin, an anti-angiogenesis effect could be significantly exhibited in a renal cancer tissue.

### <3-3> Confirmation of effect of inhibiting tumor angiogenesis according to treatment with streptonigrin and pazopanib in combination

In Example <3-2>, it was confirmed that in the case of treatment with streptonigrin which is a TGase2 inhibitor, angiogenesis around cancer cells was inhibited. Thus, it was intended to confirm whether the effect of inhibiting angiogenesis was increased during the treatment with pazopanib, among VEGF inhibitors, in combination.

Specifically, the relative ratio of the number of live cells was calculated in the same manner as in Example <2-1>, using 5µM of pazopanib and 2 nM of streptonigrin.

As a result, as illustrated in [FIG. 8], it could be confirmed that when a renal cancer cell line was treated with both pazopanib and streptonigrin in combination, the death effect on the renal cancer cell line was significantly increased as compared to when the renal cancer cell line was treated with pazopanib or streptonigrin alone, and particularly, the death effect was significantly increased in an ACHN renal cancer cell line.

### Example 4. Confirmation of effect of inhibiting tumor growth according to treatment with TGase2 inhibitor and mTOR inhibitor in combination

As confirmed in [Example 2], when cancer cells were treated with streptonigrin which is a TGase2 inhibitor and rapamycin which is an mTOR inhibitor in combination, the death effect thereof was significantly increased. Thus, when a tumor was treated with streptonigrin and rapamycin in combination, it was intended to confirm whether the growth thereof was inhibited.

Specifically, the heads of specific pathogen-free 6-week-old female BALB/c-nude mice (Seongnam, Korea) were xenografted with a CAKI-1 renal cancer cell line (5×10⁶) by subcutaneous inoculation. When the size of a tumor reached 100 mm³, the mice were treated with 0.5 mg/kg of rapamycin (intraperitoneal injection, once daily, 5 days/1 week) and/or 0.05 mg/kg of streptonigrin (oral administration, once daily1, 5 days/1 week) and bred. A solvent was used as a control. The volume of the initial tumor was measured at intervals of 3 to 4 days using a caliper), and the weight of the tumor was calculated by an equation of V = (A×B²)/2 (V = volume (mm³), A = major diameter (mm), B = minor diameter (mm)). A tumor was obtained by sacrificing the mice in a 7.5% CO₂ chamber, and photos of the obtained tumor were taken.

As a result, as illustrated in [FIG. 9], it could be confirmed that when renal cancer tumor was treated with rapamycin and streptonigrin in combination, the volume and weight of the renal cancer tumor were significantly decreased as compared to when a renal cancer tumor was treated with rapamycin or streptonigrin alone. This means that the treatment with rapamycin and streptonigrin in combination significantly inhibits the growth of a tumor.

### [Industrial Applicability]

The present invention provides a pharmaceutical composition for preventing or treating cancer by using streptonigrin which is a TGase2 inhibitor and rapamycin which is an mTOR inhibitor, a cancer treatment method, a use in the preparation of anticancer drugs and a composition to be used in cancer prevention or treatment. Further, the present invention provides a composition for inhibiting angiogenesis by using streptonigrin and a VEGF inhibitor, a method for inhibiting angiogenesis, a use in the preparation of drugs for inhibiting angiogenesis and a composition to be used in angiogenesis inhibition. Therefore, the present invention can contribute to the development of a cancer therapeutic agent and an angiogenesis inhibitor, and thus is highly industrially applicable.

## Claims

1. A pharmaceutical composition for preventing or treating cancer, comprising streptonigrin and rapamycin as active ingredients.

2. The pharmaceutical composition of claim 1, wherein the streptonigrin and rapamycin are mixed at a molar ratio of 1 : 0.01 to 1 : 100.

3. The pharmaceutical composition of claim 1, wherein the cancer is a carcinoma in which resistance to an mTOR inhibitor is induced.

4. The pharmaceutical composition of claim 3, wherein the cancer in which resistance to an mTOR inhibitor is induced is any one or more selected from the group consisting of renal cancer, breast cancer, colorectal cancer, and melanoma.

5. A pharmaceutical composition for preventing or treating cancer, further comprising an anticancer drug in the pharmaceutical composition of claim 1.

6. A composition for inhibiting angiogenesis, comprising streptonigrin and a vascular endothelial growth factor (VEGF) inhibitor as active ingredients.

7. The composition of claim 6, wherein the VEGF inhibitor is pazopanib.

8. The composition of claim 6, wherein the streptonigrin and the VEGF inhibitor are mixed at a molar ratio of 1 : 1×10⁻⁵ to 1 : 1×10⁵.

9. A pharmaceutical composition for preventing and treating cancer or inhibiting metastasis, comprising the composition of claim 6.

10. The pharmaceutical composition of claim 9, wherein the cancer is any one or more selected from the group consisting of renal cancer, breast cancer, colorectal cancer, and melanoma.

11. A method for treating cancer, the method comprising administering streptonigrin and rapamycin to a patient with cancer.

12. The method of claim 11, wherein the streptonigrin and rapamycin are mixed at a molar ratio of 1 : 0.01 to 1 : 100.

13. The method of claim 11, wherein the cancer is a carcinoma in which resistance to an mTOR inhibitor is induced.

14. The method of claim 13, wherein the cancer in which resistance to an mTOR inhibitor is induced is any one or more selected from the group consisting of renal cancer, breast cancer, colorectal cancer, and melanoma.

15. The method of claim 11, further comprising administering an anticancer drug.

16. A method for inhibiting angiogenesis, the method including treating streptonigrin and a vascular endothelial growth factor (VEGF) inhibitor.

17. The method of claim 16, wherein the VEGF inhibitor is pazopanib.

18. The method of claim 16, wherein the streptonigrin and the VEGF inhibitor are mixed at a molar ratio of 1 : 1×10⁻⁵ to 1 : 1×10⁵.

19. The method of claim 16, wherein the angiogenesis is cancer.

20. The method of claim 19, wherein the cancer is any one or more selected from the group consisting of renal cancer, breast cancer, colorectal cancer, and melanoma.

21. A use of a composition comprising streptonigrin and rapamycin in the preparation of anticancer drugs.

22. The use of claim 21, wherein the streptonigrin and rapamycin are mixed at a molar ratio of 1 : 0.01 to 1 : 100.

23. The use of claim 21, wherein the cancer is a carcinoma in which resistance to an mTOR inhibitor is induced.

24. The use of claim 23, wherein the cancer in which resistance to an mTOR inhibitor is induced is any one or more selected from the group consisting of renal cancer, breast cancer, colorectal cancer, and melanoma.

25. A use of a composition comprising streptonigrin and a vascular endothelial growth factor (VEGF) inhibitor in the preparation of drugs for inhibiting angiogenesis.

26. The use of claim 25, wherein the VEGF inhibitor is pazopanib.

27. The use of claim 25, wherein the streptonigrin and the VEGF inhibitor are mixed at a molar ratio of 1 : 1×10⁻⁵ to 1 : 1×10⁵.

28. The use of claim 25, wherein the angiogenesis is cancer.

29. The use of claim 28, wherein the cancer is any one or more selected from the group consisting of renal cancer, breast cancer, colorectal cancer, and melanoma.

30. A composition comprising streptonigrin and rapamycin to be used in cancer prevention or treatment.

31. The composition of claim 30, wherein the streptonigrin and rapamycin are mixed at a molar ratio of 1 : 0.01 to 1 : 100.

32. The composition of claim 30, wherein the cancer is a carcinoma in which resistance to an mTOR inhibitor is induced.

33. The composition of claim 32, wherein the cancer in which resistance to an mTOR inhibitor is induced is any one or more selected from the group consisting of renal cancer, breast cancer, colorectal cancer, and melanoma.

34. The composition of claim 30, further comprising an anticancer drug in the composition.

35. A composition comprising streptonigrin and a vascular endothelial growth factor (VEGF) inhibitor to be used in angiogenesis inhibition.

36. The composition of claim 35, wherein the VEGF inhibitor is pazopanib.

37. The composition of claim 35, wherein the streptonigrin and the VEGF inhibitor are mixed at a molar ratio of 1 : 1×10⁻⁵ to 1 : 1×10⁵.

38. The composition of claim 35, wherein the angiogenesis is cancer.

39. The composition of claim 38, wherein the cancer is any one or more selected from the group consisting of renal cancer, breast cancer, colorectal cancer, and melanoma.
